(19) Europäisches Patentamt
European Patent Office
Office européen des brevets

(11) Publication number: **0 030 516**
**B1**

(12) **EUROPEAN PATENT SPECIFICATION**

(45) Date of publication of patent specification: **09.02.83**

(21) Application number: **80630056.2**

(22) Date of filing: **28.11.80**

(51) Int. Cl.³: **C 07 C 19/02,**
C 07 C 17/16, B 01 J 31/06
//C08F8/40

(54) **Process for preparing primary alkyl chlorides.**

(30) Priority: **03.12.79 US 99307**
**22.08.80 US 180189**

(43) Date of publication of application:
**17.06.81 Bulletin 81/24**

(45) Publication of the grant of the patent:
**09.02.83 Bulletin 83/6**

(84) Designated Contracting States:
**BE DE FR GB IT NL SE**

(56) References cited:
**US - A - 3 708 462**

**SYNTHESIS, January 1974, STUTTGART (DE), D. LANDINI et al.: "Conversion of primary alcohols to alkyl chlorides using aqueous hydrochloric acid in the presence of phase-transfer catalysts", pages 37—38**

(73) Proprietor: **THE GOODYEAR TIRE & RUBBER COMPANY**
**1144 East Market Street**
**Akron, Ohio 44316 (US)**

(72) Inventor: **Parker, Dane K.**
**945 Norwich Avenue**
**North West Massillon, Ohio 44646 (US)**

(74) Representative: **Weyland, Joseph Jean Pierre et al,**
**Goodyear International Tire Technical Center**
**Patent Department Avenue Gordon Smith**
**L-7750 Colmar-Berg (LU)**

**EP 0 030 516 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing primary alkyl chlorides

This is a continuation-in-part of Application Serial Number 099307, filed December 3, 1979.

*Technical Field*

Primary alkyl alcohols can be converted to the corresponding alkyl chlorides in the presence of aqueous hydrochloric acid and a quaternary phosphonium salt, the improvement characterized in that an organic resin bound or triphase quaternary phosphonium salt is used as the catalyst.

*Background Art*

The classical conversion of primary alcohols to alkyl chlorides requires the use of hydrogen chloride gas and the presence of catalytic amounts of zinc chloride. See M. T. Atwood, *The Journal of the American Oil Chemists Society,* vol. 40, page 64, (Feb. 1963) and R. Stroh, W. Hahn and Houben-Weyl, *Methoden der Organischen Chemie,* 4th ed. E. Muller, Ed., Vol. V/3, Georg Thieme Verlag Stuttgart, 1962, p. 831. The references disclose that use of aqueous hydrochloric acid is less suitable since anhydrous reaction conditions are preferred. These methods give poor yields and require the use of a large amount of zinc chloride. See A. Guyer, A. Bieler, and E. Hardmeyer, *Helv. Chim. Acta 20,* 1463 (1937).

Landini, Montanari a:ıd Rolla in *Synthesis,* January 1974, page 37 reported that the problems of poor yield and excessive amounts of reactants can be overcome when the reaction is carried out in the presence of a phase-transfer catalyst in a heterogenous system. The following equation is indicative of Landini et al's reaction:

$$n\text{—}C_n H_{2n+1} OH + Aq.\ HCl \xrightarrow[\Delta]{C_{16}H_{33}P^{\oplus} (C_4H_9)_3Br^{\ominus}} n\text{—}C_nH_{2n+1} Cl + H_2O$$

Prior to the advent of the phase-transfer catalyst concept the general order of reactivity of alkyl halides was normally RI > RBr > RCl. Unfortunately this order of reactivity is just the reverse of their economic desirability. This fact has led to the very limited industrial applications of alkyl halides.

Recently using the phase-transfer concept it has been discovered that the order of reactivity of the alkyl halides is RCl > RBr > RI in some cases.

It has been reported that when water insoluble primary alcohols are reacted with aqueous concentrated hydrochloric acid at 105°C. in the presence of catalytic quantities of hexadecyltributylphosphonium bromide, the conversion into corresponding primary alkyl chlorides averaged 60% after 8 hours and reached 90 and 95% after 30 and 45 hours respectively. Landini et al, *Synthesis,* page 37, supra, determined that the chain length of the primary alkyl alcohol had no noticeable effect on the rate of conversion in the series of alcohols from 6 to 16 carbon atoms. It was found both yields and reaction rates drastically dropped in the case of water-soluble alcohols. Additionally, it was determined that the reaction proceeded without the synthesis of the undesired by-products of dialkyl ethers or isomeric chlorides.

The process of the present invention unlike some cited references does not require the use of anhydrous HCl gas. By use of the process of this invention high yields of the primary alkyl chlorides can be obtained by using aqueous concentrated hydrochloric acid. The crude alkyl chlorides then may be used directly to prepare many valuable products, for example, sulfides, disulfides, esters, ethers, nitriles and etc. Industrial production of alkyl chlorides would preferably be conducted on a continuous basis due to economic benefits over batch preparations.

*Disclosure of Invention*

In a process for the conversion of primary alkyl alcohols which have limited water solubility to the corresponding alkyl chlorides in the presence of aqueous hydrochloric acid and a catalyst, the improvement characterized in that an organic resin bound or triphase catalyst prepared from lightly crosslinked (1 or 2% crosslink density) polystyrene resin containing pendant halogen functionality by a quaternization reaction with a tertiary phosphine as illustrated by the structural formula:

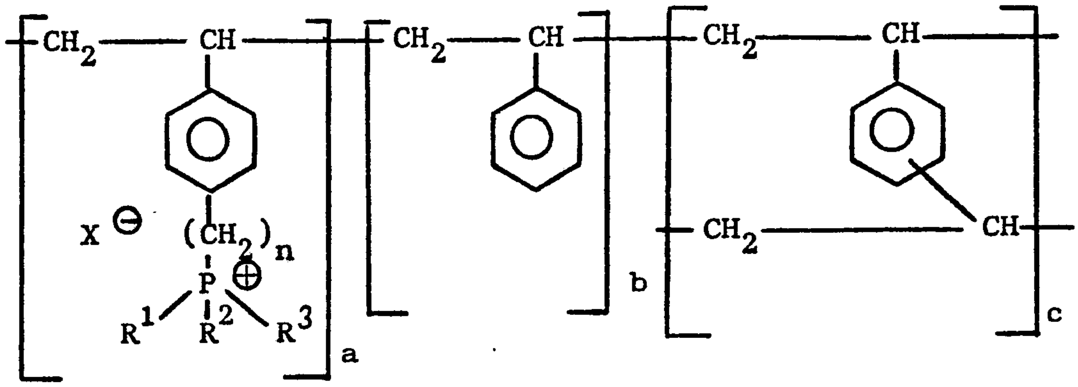

wherein the a, b, c ratio may vary between 1/98/1 to 99/0/1, the more preferred range being 1/98/1 to 65/34/1 with the most preferred range 5/94/1 to 30/69/1, and wherein $R^1$, $R^2$ and $R^3$ are the same or different radicals selected from the group comprised of alkyls of 1 to 12 carbon atoms, secondary alkyls of 3 to 12 carbon atoms, cycloalkyls of 5 to 8 carbon atoms; $X^{\ominus}$ is a halide radical selected from the group consisting of $F^{\ominus}$, $Br^{\ominus}$, $Cl^{\ominus}$ or hydrogen sulfate ion $HSO_4^{\ominus}$, and wherein n equals 1 to 12; is used.

*More Detailed Description of Disclosure*

Representative of the alcohol starting materials used in this process are alcohols such as 1-pentanol, 1-hexanol, 1-heptanol, 1-octanol, 1-nonanol, 1-decanol, 3-methyl-1-butanol, 3,3-dimethyl-1-butanol and 4-methyl-1-pentanol.

Representative of the products formed by the process of this invention are alkyl chlorides such as 1-chloropentane, 1-chloroheptane, 1-chlorooctane, 1-chlorononane, 1-chlorodecane, 3-methyl-1-chlorobutane, 3,3-dimethyl-1-chlorobutane and 4-methyl-1-chloropentane. The process of this invention is continuous as the alkyl chloride product is removed by distillation of the alkyl chloride azeotrope with water.

It has been determined that the phosphonium ions are somewhat more effective and thermally stable than the corresponding ammonium catalyst in the process of this invention. It has also been determined that, in general, the longer the alkyl chain length of the catalyst the greater the efficiency of the catalyst.

The process of this invention is limited to organic resin bound or the so-called "triphase" catalyst system. These polymer-bound catalysts may be easily prepared from lightly crosslinked (1 or 2% crosslink density) polystyrene resins containing pendent halogen functionality by a quaternization reaction with a tertiary phosphine as illustrated by the following formula:

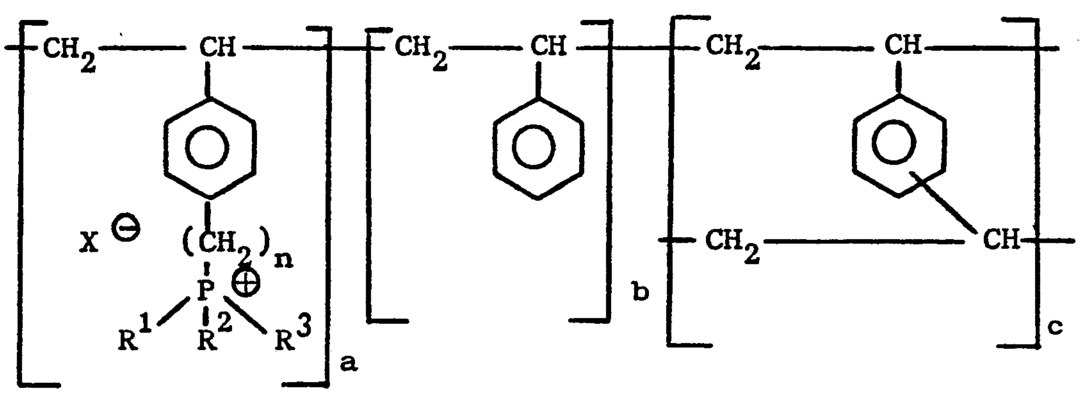

wherein the a, b, c ratio may vary between 1/98/1 to 99/0/1, the more preferred range being 1/98/1 to 65/34/1 with the most preferred range 5/94/1 to 30/69/1, and wherein $R^1$, $R^2$, $R^3$ are the same or different radicals selected from the group comprised of alkyls of 1 to 12 carbon atoms, secondary alkyls of 3 to 12 carbon atoms, cycloalkyls of 5 to 8 carbon atoms. $X^{\ominus}$ is a halide radical selected from the group consisting of $F^{\ominus}$, $Br^{\ominus}$, $Cl^{\ominus}$ or hydrogen sulfate ion $HSO_4^{\ominus}$, and wherein n equals 1 to 12.

As an example, polymer bound benzyl-tri-n-butyl phosphonium chloride is prepared according to the following procedure:

A 2 liter 3 neck flask equipped with a mechanical stirrer, condenser, thermometer and nitrogen gas inlet was charged with 103 grams (0.124 moles chloride content) of chloromethylated 1% crosslinked polystyrene resin (marketed under the trade name Bio-Beads SX—1). The resin was then swollen by the addition of 1 liter dimethylformamide. The system was purged with a stream of nitrogen and 32.4 grams (.160 moles) tri-n-butyl phosphine was added. The mixture was brought to

120—130°C. with stirring for four hours. The mixture was then cooled to 70°C., filtered warm and subsequently washed with dimethylformamide three times and with acetone four times. The resin was dried in a vacuum oven at 80—100°C. providing the catalyst resin in a yield of 131.3 grams.

*Best Mode For Carrying Out The Invention*

The overall reaction may be represented by the following equation:

$$C_nH_{(2n+1)}OH+HCl \xrightarrow[\Delta]{\text{Cat.}} CnH_{(2n+1)}Cl+H_2O$$

wherein $5 \leqslant n \leqslant 10$.

As little as 1 mol% of phosphonium salt catalyst per mole of alcohol may be used, however, due to the stability of the catalyst under the reaction conditions, it is advantageous and desirable to increase the catalyst level to 10, 20, 50 or more mole percent per mole of alcohol to increase the rate of conversion of alcohol of chloride. As little as 1.5 moles of concentrated 37% aqueous hydrochloric acid per mole of alcohol in the reaction vessel can be used, however, it is advantageous to use from 5 to 20 moles of aqueous hydrochloric acid per mole of alcohol to speed conversion.

Provided to illustrate, but not limit the scope of the present invention is the following example:

Example I

Preparation of 1-Chlorohexane Using Polymeric Phosphonium Catalysts

*Preparation of Catalyst*

To a one liter flask was added 50 grams of 1% crosslinked chloromethylated polystyrene resin containing 1.24 meq. chlorine per gram resin, 500 milliliters of dimethylformamide and 16.2 grams (0.08 moles) of tributylphosphine. The mixture was heated with stirring for four hours at 120 to 130°C. The mixture was cooled and filtered and the product was washed with dimethylformamide 3 times and then with acetone 3 times. The product was dried in a vacuum oven at 100°C. yielding 69.4 grams.

*Preparation of 1-Chlorohexane*

Of the phosphonium resin just prepared 32.3 grams of the phosphonium resin containing 2.03 moles phosphorus was added to 103 grams (1 mole) of n-hexyl alcohol in a 2 liter flask. 500 milliliters of concentrated hydrochloric acid and a few boiling chips were added to the mixture. A condenser was attached and the mixture refluxed for 43 hours under slow stirring. The mixture was cooled and the resin catalyst was filtered off. The organic layer was separated from the filtrate in a separation funnel yielding 54 grams of a pale yellow liquid. The resin was then washed with a mixture of water and $CH_2Cl_2$ as a reslurry. The lower organic layer was separated from the filtrate and the $CH_2Cl_2$ was distilled off using a hot water bath at approximately 50°C. There remained a residue of 59.1 grams which was shown by gas chromatography to be 1-chlorohexane. The total yield was 113.1 grams (.94 moles) or 93.7% of theory.

*Industrial Applicability*

The process of the present invention would greatly enhance the industrial use of alkyl chlorides since the tri-phase catalysts will greatly lessen the cost of production. In and of themselves alkyl chlorides can be used in numerous ways, however, the major value of these alkyl chlorides is their ability to be used as intermediates in the preparation of many materials.

It is evident that the process of this invention would greatly enhance the productivity of alkyl chlorides in that the reaction proceeds yielding products in good yield and good purity.

It is believed that because of the tri-phase catalyst systems, the use of the alkylchlorides will become more widespread as industry accepts and develops this technique.

While certain representative embodiments and details have been shown for the purpose of illustrating the invention, it will be apparent to those skilled in this art that various changes and modifications may be made therein without departing from the scope of the invention.

**Claims**

1. In a process for conversion of primary alkyl alcohols to the corresponding alkyl chlorides in the presence of aqueous hydrochloric acid and a catalyst, the improvement characterized in that an organic resin bound or triphase catalyst prepared from lightly crosslinked (1 or 2% crosslink density) polystyrene resins containing pendant halogen functionality by a quaternization reaction with tertiary phosphine as illustrated by the structural formula:

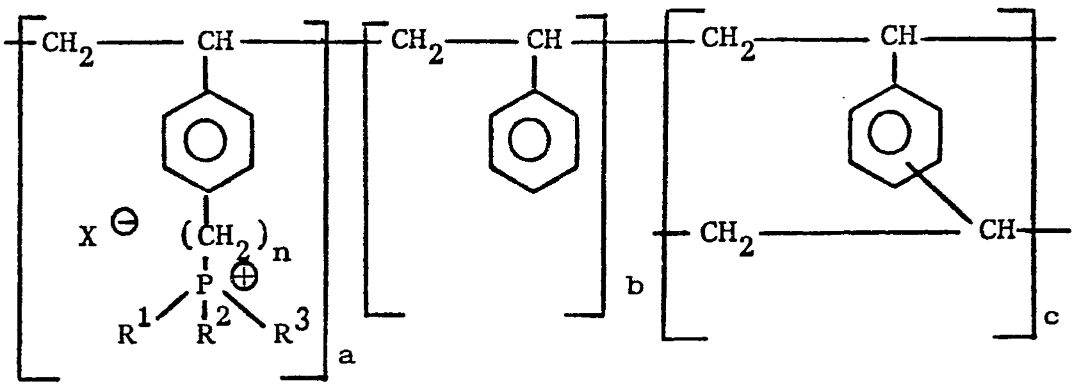

wherein the a, b, c ratio may vary between 1/98/1 to 99/0/1, the more preferred range being 1/98/1 to 65/34/1 with the most preferred range 5/94/1 to 30/69/1, and wherein $R^1$, $R^2$, $R^3$ are the same or different radicals selected from the group comprised of alkyls of 1 to 12 carbon atoms, secondary alkyls of 3 to 12 carbon atoms, cycloalkyls of 5 to 8 carbon atoms; $X^{\ominus}$ is a halide radical selected from the group consisting of $F^{\ominus}$, $Br^{\ominus}$, $Cl^{\ominus}$ or hydrogen sulfate ion $HSO_4^{\ominus}$, and wherein n equals 1 to 12; is used.

2. A process according to claim 1 wherein the primary alkyl alcohol is selected from the group consisting of 1-pentanol, 1-hexanol, 1-heptanol, 1-octanol, 1-nonanol, 1-decanol, 3-methyl-1-butanol, 3,3-dimethyl-1-butanol, 4-methyl-1-pentanol.

**Patentansprüche**

1. Verfahren zur Umwandlung von primären Alkylaloholen in die entsprechenden Alkylchloride in Gegenwart von wäßriger Chlorwasserstoffsäure und eines Katalysators, dadurch gekennzeichnet, daß ein an ein organisches Harz gebundener oder Dreiphasenkatalysator, hergestellt aus leicht vernetzten (1 oder 2% Vernetzungsdichte) Polystyrolharzen die eine hängende Halogenfunktionalität aufweisen, durch eine Quarternisierungsreaktion mit einem tertiären Phosphin der Strukturformel

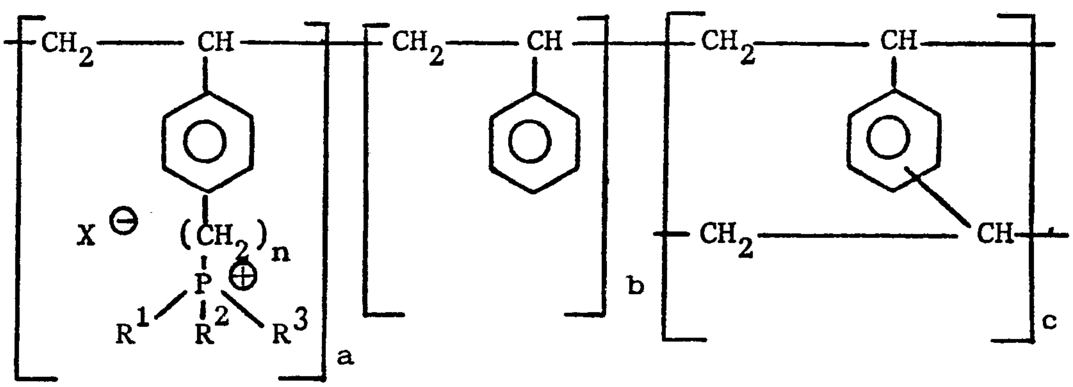

worin das Verhältnis a, b, c zwischen 1/98/1 und 99/0/1 schwanken kann und der bevorzugtere Bereich zwischen 1/98/1 und 65/34/1 liegt und der bevorzugteste Bereich zwischen 5/94/1 und 30/69/1 variiert, und wobei die Substituenten $R^1$, $R^2$ und $R^3$ gleich oder verschiedene Reste sind, ausgewählt aus der Gruppe, die aus Alkylen mit 1 bis 12 Kohlenstoffatomen, sekundären Alkylen mit 3 bis 12 Kohlenstoffatomen und Cycloalkylen mit 5 bis 8 Kohlenstoffatomen besteht, $X^{\ominus}$ ein Halogenidrest ist, ausgewählt aus der Gruppe, die aus $F^{\ominus}$, $Br^{\ominus}$, $Cl^{\ominus}$ oder Hydrogensulfationen $HSO_4^{\ominus}$ besteht, und wobei n 1 bis 12 ist, verwendet wird.

2. Verfahren nach Anspruch 1, dadurch gekennzeichnet, daß der eingesetzte primäre Alkohol aus der Gruppe ausgewählt wird, die aus 1-Pentanol, 1-Hexanol, 1-Heptanol, 1-Octanol, 1-Nonanol, 1-Decanol, 3-Methyl-1-butanol, 3,3-Dimethyl-1-butanol sowie 4-Methyl-1-pentanol besteht.

**Revendications**

1. Dans un procédé de transformation d'alcools alkyliques primaires en chlorures d'alkyle correspondants en présence d'acide chlorhydrique aqueux et d'un catalyseur, le perfectionnement caractérisé en ce qu'on utilise un catalyseur à trois phases ou fixé à une résine organique, ce catalyseur étant préparé à partir de résines de polystyrène légèrement réticulées (densité de réticulation: 1 ou 2%) contenant une fonctionnalité halogène latérale, par une réaction de quaternisation avec une phosphine tertiaire comme illustré par la formule structurale:

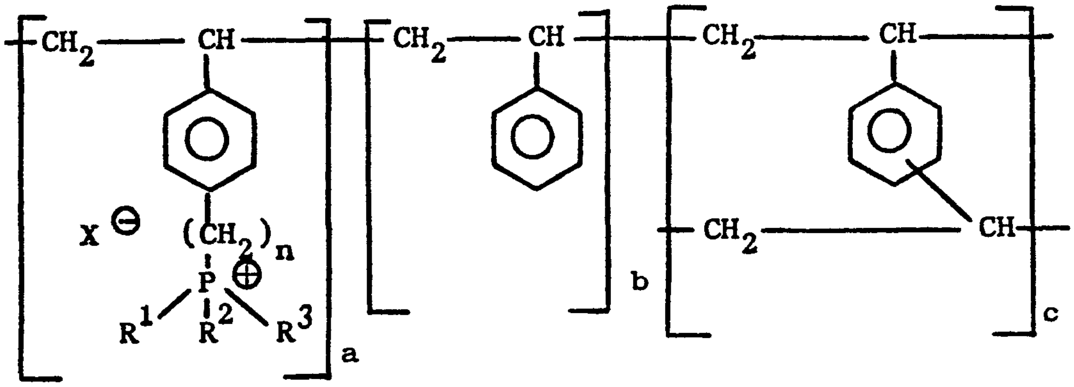

dans laquelle le rapport a, b, c peut varier entre 1/98/1 et 99/0/1, l'intervalle davantage préféré étant compris entre 1/98/1 et 65/34/1, l'intervalle de loin préféré étant compris entre 5/94/1 et 30/69/1, tandis que $R^1$, $R^2$ et $R^3$ représentent des radicaux identiques ou différents choisis parmi le groupe comprenant des groupes alkyle contenant 1 à 12 atomes de carbone, des groupes alkyle secondaires contenant 3 à 12 atomes de carbone et des groupes cycloalkyle contenant 5 à 8 atomes de carbone; $X^{\ominus}$ représente un radical halogénure choisi parmi le groupe comprenant $F^{\ominus}$, $Br^{\ominus}$, $Cl^{\ominus}$ ou l'ion sulfate d'hydrogène $HSO_4^{\ominus}$, n étant un nombre de 1 à 12.

2. Procédé suivant la revendication 1, caractérisé en ce que l'alcool alkylique primaire est choisi parmi le groupe comprenant le 1-pentanol, le 1-hexanol, le 1-heptanol, le 1-octanol, le 1-nonanol, le 1-décanol, le 3-méthyl-1-butanol, le 3,3-diméthyl-1-butanol et le 4-méthyl-1-pentanol.